# EUROPEAN PATENT APPLICATION

(11) **EP 2 426 497 A1**
(43) Date of publication of application: **07.03.2012**
(21) Application number: 10175083.4
(22) Date of filing: 02.09.2010
(51) Int. Cl.: G01N 33/543, B01L 3/00

(54) **Generation and use of liquid stacks in microchannels or capillaries**

(71) Applicant: Ludwig-Maximilians-Universität München, 80539 München (DE)
(72) Inventor: Braun, Dieter, 83661, Lenggries (DE); Wolfertstetter, Fabian, 81675, München (DE)
(74) Representative: Lucke, Andreas

(57) **Abstract**

Disclosed herein is a method of stacking at least two different liquids (30a, 30b) in a capillary (14). According to the method, a first liquid (30a) and a second liquid (30b) are filled into the capillary (14). Next, a gas volume trapped between the first and second liquids (30a, 30b) is removed by exposing said gas volume (32) to a force biasing the gas volume (32) along the longitudinal axis of the capillary (14), and generating a pressure inside the capillary (14) suitable to compress said gas volume (32), such that the compressed gas volume (32) is moved through one of the first and second liquids (30a, 30b) in response to said force. Further disclosed is a method of bringing a carrier in surface contact with at least two different liquids (30a, 30b), in which at least first and second liquids (30a, 30b) are stacked as described above and the carrier is moved along the longitudinal axis of the capillary (14) through said at least two liquids. A corresponding apparatus for carrying out this method is also disclosed.

## Description

### FIELD OF THE INVENTION

The present invention relates to the field of biochemical, medical or pharmaceutical analysis. In particular, the invention may be employed in the field of biochemical, medical or pharmaceutical assays.

### BACKGROUND OF THE INVENTION

An immunoassay is a biochemical test that measures the presence or concentration of an analyte in a biological liquid such as a serum or urine. The basic idea behind an immunoassay is the recognition of an analyte in a liquid phase by observing the binding of an antigen and an antibody. Herein, either one of the antigen and antibody could be the analyte to be detected.

A very powerful immunoassay technique is based on a mixture of different populations of so-called microspheres or microbeads. Each of these populations is defined by an individual fluorescence color (for example different hues of red) and a coating with a specific capture reagent or receptor. When the microspheres are incubated by immersing them in a fluid sample, for example a serum or urine, each microsphere population binds a target molecule, i.e. analyte, with the corresponding receptor attached to the microsphere. For example, if the target molecule is a specific antibody, the suitable receptor would be the corresponding antigen. The analytes that are bound to the surface of the microspheres can then be colored with a specific fluorescence marker, for example a green fluorescence marker, which is also referred to as a conjugate.

The microspheres can then be automatically analyzed with respect to the microsphere classification based on red fluorescence light and the quantification of the bound analytes by the green fluorescence. The results of this analysis can be exploited entirely automatically by commercially available analyzing apparatuses.

While this analysis method is extremely powerful, unfortunately, the preparation of the different populations of microspheres is rather involved. Typically, for attaching specific capture reagents at the microsphere surface, as many as 20 processing steps are necessary, in which as many as six or even more liquids have to be brought in contact with the microspheres. Accordingly, the handling of the microspheres such as to expose them to a large number of different fluids tends to be quite demanding. If the process is carried out manually, the procedure is very time consuming. On the other hand, an entirely automatical process calls for a very complicated and expensive apparatus.

The present invention was motivated by the idea of facilitating the process of preparing such microspheres, but it is to be noted that the inventive solution described below is not limited to this application.

It was recognized that the main problem in the prior art was the transport of the microspheres between the different liquids that the microsphere has to be exposed to in generating the desired coating. It was conceived that instead of moving the microspheres between different containers with different liquids, it would be attractive to move the microspheres through different liquids stacked on top of each other in a single container. Clearly, this concept would require some technique to "stack" different liquids in the same container in a way that rather sharp boundaries between the different liquids are formed and that a mixture of adjacent liquids on the time scale of the process is prevented.

The inventors tried to stack different liquids in a very thin capillary having a diameter of one millimeter or less. The advantage of a thin capillary is two-fold. First of all, with a small diameter, the mixture of the adjacent liquids due to diffusion can be kept rather low. Second, with the small diameters, a comparatively small volume of the liquid can be combined with a rather long extension in axial direction of the capillary, which allows to combine an efficient use of adjacent liquids with a moderate mixture of the liquids at least in a central portion of each liquid layer.

Unfortunately, it turned out to be very difficult to form the stack of different liquids in a capillary. If two different liquids are drawn into the capillary with no air gap inbetween, due to the laminar flow profile of the liquid inside the capillary induced by friction of the liquid at the capillary walls, the liquid-liquid interface tends to wash out significantly while advancing inside the capillary. This mixing of the two liquids can be prevented by separating the two liquids by a gas volume, which eventually has to be removed. However, due to the capillary attraction of the liquid to the capillary wall, an air volume trapped between two liquids in the capillary turned out to be difficult to remove.

US 7,655,470 B2 discusses how a gas bubble trapped between two liquid plugs can be removed. According to this document, it is suggested to apply a pressure on either the side of the plugs and to use a microchannel that is permeable to gas, so that the gas can be purged out. However, this prior art also teaches that if the microchannel is not permeable to gas, for example a glass capillary, the merger of two plugs would not work.

The problem underlying the invention is thus to provide a method of bringing a carrier, such as a microsphere or microbead in surface contact with at least two different liquids in an easy and efficient manner. A further problem underlying the invention is to provide a method of stacking at least two different liquids in a microchannel or capillary, so that a direct boundary between the two liquids may be found.

### SUMMARY OF THE INVENTION

This problem is solved by a method of stacking at least two different liquids in a microchannel or capillary according to claim 1 and a method of bringing a carrier in surface contact with at least two different liquids according to claim 7. Further, this solution may be employed in an apparatus according to claim 12. Preferable embodiments are defined in the dependent claims.

According to the invention, a method of stacking at least two different liquids in a microchannel or capillary comprises the following steps:
- consecutively filling a first liquid and a second liquid into the microchannel or capillary and,
- a step of removing a gas volume trapped between the first and second liquids by
   - exposing said gas volume to a force biasing said gas volume along the longitudinal axis of said microchannel or capillary, and
   - generating a pressure inside said microchannel or capillary suitable to compress said gas volume such that the compressed gas volume is moved through one of said first and second liquids in response to said force.

In the easiest case, the aforementioned force biasing the gas volume along the longitudinal axis may simply be the hydrostatic lift experienced by the gas volume if the microchannel or capillary is placed in an upright position, i.e. if the longitudinal axis thereof is in a vertical position or has at least a vertical component.

It has been confirmed by the inventors that by applying a suitable pressure inside the microchannel or capillary, the trapped gas volume may be compressed to a size where its diameter decreases below the diameter of the microchannel or capillary. Along with the decrease of the trapped gas volume, the capillary wall in the region of the gas volume will be increasingly wetted by the first and second liquids, until all of the capillary wall is wetted by either one of the liquids. At this point, the formerly trapped gas volume is compressed to a bubble that can escape through one of the first and second liquids, typically driven by the hydrostatic lift.

It has been found that this method is extremely reliable and easy to carry out and is particularly suitable for automatization. It was further found that this method is a very gentle way to remove the gas trapped between the two liquids, without any substantial perturbation and mixing of the two liquids, allowing for a very sharp an clean boundary between the first and second liquids.

Preferably, at least one of said first and second liquids comprises a solution containing receptors for use in biochemical, medical or pharmaceutical assays, and in particular antigens or antibodies for use in immunoassays.

Accordingly, the stack of liquids generated can be used in the process of covering carriers such as microbeads or microspheres with suitable capture reagents. While the invention is particularly useful in preparing microspheres for immunoassays, it may equally be used to process carriers for use in other types of assays, in particular DNA assays, protein assays, RNA assays and the like.

In a preferred embodiment the stack of liquids may also be used for conducting the assay with a certain sample. In this case, at least one of the first and second liquids could be a liquid comprising an analyte for detection in biochemical, medical or pharmaceutical assays, and/or at least one of said first and second liquids may comprise a washing solution or a solution comprising conjugate markers.

Note that these are only two exemplary uses of liquid stacks that may be generated by the method of the invention. It is however understood that the invention is not limited in this regard and that the method of the invention can be employed in any situation where stacked liquids are desired.

Preferably, the capillary or microchannel has an inner diameter of 300 µm - 1 mm, preferably 400 µm to 800 µm. These small diameters allow to keep a mixture of adjacent liquids by diffusion low on useful timescales and to also keep the amount of liquids needed small. It has been confirmed in experiments that even with these narrow microchannels or capillaries, the method of removing trapped gas between two adjacent liquid plugs is entirely functional.

Preferably, the longitudinal length of the volume of the first and/or second liquid is at least 1, preferably at least 5 mm. With these longitudinal extensions of the liquid layers, a mixture by diffusion can be kept low for useful timescales at least in a central portion of the liquid layer.

The pressure generated inside the microchannel or a capillary is preferably between 0.5 - 1 MPa.

According to a further aspect of the invention, a method of bringing a carrier and in particular a microbead or microsphere in surface contact with at least two different liquids is provided. In this method, at least first and second liquids are preferably stacked in a microchannel or capillary according to one of the preferred embodiments discussed above. Further, the method comprises a step of moving the carrier along the longitudinal axis of the microchannel or capillary through said at least two liquids. This way, the carrier can be easily and efficiently exposed to two or more different liquids without having to move the carrier between different containers.

Note that this novel method of bringing a carrier, in particular a microbead or microsphere in surface contact with at least two different liquids can be employed irrespectively of the specific way the liquid stack is generated, i.e. be it according to one of the above described embodiments or in any other suitable way. For example, it is possible to generate a stack of liquids drawn into the capillary without air inbetween but with a thin gel layer separating each pair of adjacent liquids and preventing a mixture of the adjacent liquids upon drawing them into the capillary. In this embodiment, the gel would remain between adjacent liquids. Carriers would be chosen small enough such as to be able to pass through the gel.

Preferably, the movement of the carrier along the axis of the microchannel or capillary is controlled by applying a force to said carrier, and in particular by applying one or more of an electromagnetic force, an electrophoretic force, a magnetic force or a force obtained by optical tweezers.

In an optical tweezers-based embodiment the particles are kept in the waist of a laser beam with a gaussian intensity profile. When moving either the laser beam along the capillary or vice versa, the particle always stays in the middle of the laser beam and thus moves relative to the liquid stacks.

Using electrophoresis one would use charged particles that move along an electrical field applied along the capillary.

In a simple embodiment, the force is the gravitational force that makes the particles sink at a predetermined speed in each liquid phase. By adjusting the longitudinal length of each phase, the exposure times of the particle to each phase can then be controlled.

In an especially preferred embodiment, the carrier comprises a paramagnetic material and/or the electromagnetic force on the carrier is generated by means of a magnetic field, which has a local maximum of field strength inside the microchannel or capillary, wherein said local maximum is movable along the longitudinal axis thereof. A paramagnetic material is attracted to regions of higher magnetic field strengths. Consequently, if a magnetic field having a local maximum of field strength is formed inside the microchannel or capillary, the paramagnetic carrier will be attracted to the region of the local maximum. Then, if the local maximum of the field strength is moved along the longitudinal axis of the microchannel or capillary, the paramagnetic carrier will follow this motion. This is a very easy and reliable way of moving the carrier through the liquid in a controlled manner. In one embodiment, the movable magnetic field maximum is obtained by providing a moving magnet. In the alternative, the moving magnetic field maximum may also be obtained by modulating the magnetic field generated by a stationary magnetic assembly.

Preferably, the velocity of the movement of the carrier with respect to said liquids is controlled such that the surface contact to each of the liquids is sustained for predefined times.

Further, the inventors have recognized that the inventive method of stacking liquids in a microchannel or capillary, as well as the method of bringing a carrier in surface contact with liquids in such a stack is particularly suitable for automatization. This is one of the reasons why it is believed that the invention will find very favorable use in biochemical, medical or pharmaceutical analysis, where a constant trend for automatization is observed.

Accordingly, one further aspect of the invention is directed to an apparatus for bringing a carrier in surface contact consecutively with at least two liquids. The apparatus comprises means for filling a first liquid and a second liquid into a microchannel or capillary, means for generating a pressure inside said microchannel or capillary suitable for compressing a gas volume trapped between said first and second liquids such that said compressed gas volume is moved through one of said at least two liquids and means for applying a force to said carrier such as to move said carrier through said at least two liquids.

Preferably, the apparatus comprises a control unit for carrying out a method of stacking at least two different liquids in a microchannel or capillary, and/or a method of bringing a carrier in surface contact with at least two different liquids according to one of the embodiments summarized above.

Preferably, the means for applying a force to said carrier comprises a solenoid adapted to receive an electrical current and containing a bore in which the microchannel or capillary is received. The solenoid is moveable in longitudinal direction of said microchannel or capillary. According to this embodiment, the solenoid will generate a maximum magnetic field strength in a central portion of the bore and thus within the microchannel or capillary. Consequently, paramagnetic carriers will be attracted to this maximum field strength region. Then, if the solenoid is moved in longitudinal direction of the microchannel or capillary, the paramagnetic carrier will accompany this motion. Consequently, the paramagnetic carriers can be moved in a controlled manner along the longitudinal axis of the microchannel or capillary and hence through the different liquids stacked therein.

### BRIEF DESCRIPTION OF THE FIGURES

- Fig. 1: is a schematic view of an apparatus for bringing a carrier in surface contact consecutively with at least two liquids, and
- Fig. 2: is a sequence of schematic snapshots showing the method of removing a gas volume trapped between two liquids in a capillary.

### DESCRIPTION OF THE PREFERRED EMBODIMENT

For the purposes of promoting an understanding of the principles of the invention, reference will now be made to the preferred embodiment illustrated in the drawings and specific language will be used to describe the same. It will nevertheless be understood that no limitation of the scope of the invention is thereby intended, such alterations and further modifications in the illustrated device and method and such further applications of the principles of the invention as illustrated therein being contemplated therein as would normally occur now or in the future to one skilled in the art to which the invention relates.

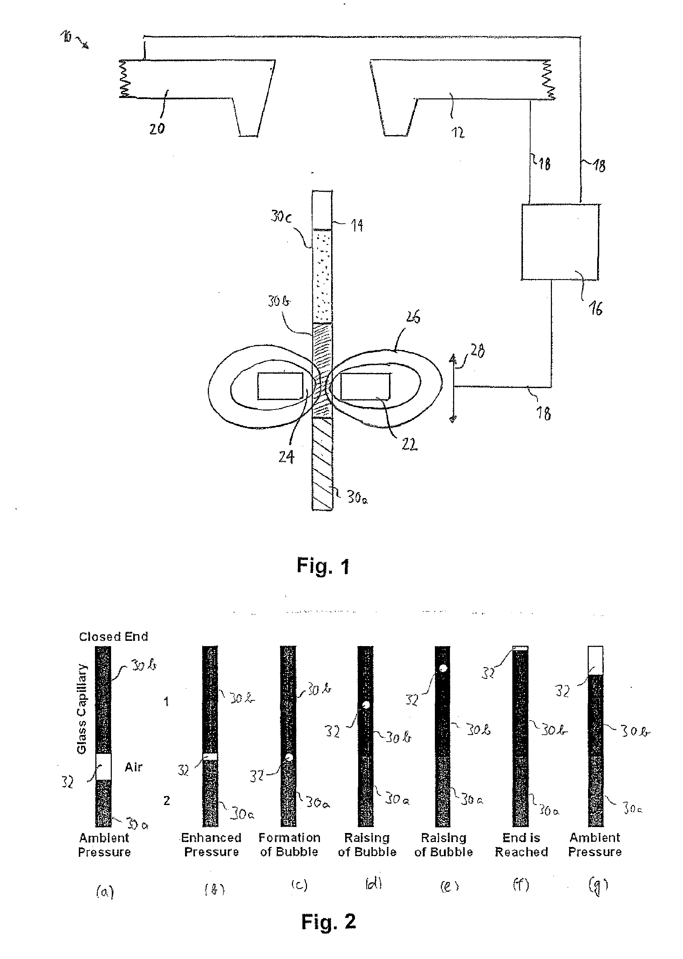

Fig. 1 is a highly schematic diagram of an apparatus 10 for bringing a carrier in surface contact consecutively with at least two liquids. In the exemplary embodiment shown, the carriers are microspheres that are to be processed such as to attach predetermined capture reagents to the surface thereof, such as antigen for use in immunoassays.

The apparatus 10 comprises automatic filling means 12 for filling, typically drawing a liquid into a capillary 14 by subatmospheric pressure. The filling means 12 can for example be formed by a laboratory robot under the control of a control unit 16 connected with the filling device 12 via a signal line 18.

The apparatus 10 further comprises a pressurizing device 20 that can be moved under the control of the control unit 16 to the upper end of capillary 14 for engagement therewith and is adapted to pressurize the interior of the capillary 14. Alternatively, the filling means 12 could be provided with a precision pump that is capable of generating both, the subatmospheric pressure for drawing or dragging the liquid stacks into the capillary 19 and to also generate the high pressure to make the gas bubble ascent inside the capillary, with the opposite end of the capillary sealed in a suitable way. A number of further alternative embodiments are conceivable, e.g. an embodiment employing a pressure chamber, and the invention is not limited to any of these.

The apparatus 10 further comprises a solenoid 22 having a bore 24 in which the capillary 14 is received. Also, in Fig. 1, a magnetic field 26 generated by the solenoid 22 is schematically represented by magnetic field lines. The solenoid 22 can be moved along the longitudinal direction of the capillary 14 by means of a moving device 28, which in Fig. 1 is symbolically represented by a double arrow. The moving device is operated under control of the control unit 16 as well.

In Fig. 1, a stack of three different liquids 30a, 30b and 30c is shown, which are symbolically represented by different types of filling.

Next, the operation of the apparatus 10 of Fig. 1 is described.

To begin, the stack of liquids 30a to 30c is to be formed inside the capillary 14. For this purpose, a filling device 12 is moved to the top of the capillary 14 under the control of control unit 16, and the first liquid 30a is filled, typically drawn into the capillary. Next, the filling device 12 is used to fill the middle section of the capillary 14 with a volume of the second liquid 30b with a small portion of air or bulk gas inbetween separating the first and second liquids and avoiding a mixture of the first and second liquids 30a, 30b. This procedure is repeated until a desired number of liquids are stacked (three in the shown embodiments), each separated by a gas volume. Since the capillary is very narrow, having a diameter of for example 500 µm or 800 µm, the air volumes will be trapped, as is specifically shown for the gas volume between the first and second liquids 30a, 30b under reference sign 32 in Fig. 2(a).

In order to remove the air volume 32 , the pressurizing device 20 is moved to the top of capillary 14 and attached thereto under the control of control unit 16. Also, the bottom of the capillary 14 is sealed by a suitable sealing device (not shown). Then, the pressure inside the capillary 14 is gradually increased by the pressuring device 20 and consequently, the air volume 32 will be compressed, as is shown in Fig. 2(b). Eventually, the air volume 32 will be compressed such as to form a bubble as shown in Fig. 2(c) which has no contact with the inner walls of the capillary 14 anymore, so that the entire inner wall of capillary 14 is wetted by one of liquids 30a and 30b. At this time, the bubble starts rising to the top due to the hydrostatic lift, as is shown in Figs. 2(d) and 2(e). Note that the third liquid 30c is not shown in Fig. 2 for simplicity. An air volume trapped between the second and third liquids 30b, 30c would behave just the same as the air volume 32 and ascend simultaneously with it through the third liquid 30c.

Fig. 2(f) shows a case where the bubble has reached the upper end of the capillary 14 but is still pressurized. Fig. 2(g) shows the same situation with the pressure being released to ambient pressure, where the air volume 32 has expanded again.

It has been confirmed by the inventors that the way of removing the air volume 32 trapped between the two liquids 30a and 30b can be quite easily and reliably performed by applying pressures in the range of for example 0.5 - 1 MPa, which can be generated by the pressurizing device 20 without great technical difficulties.

By applying this method of removing trapped air 32 for all air volumes trapped between each adjacent liquids 30a to 30c simultaneously, a liquid stack as shown in Fig. 1 can be generated.

Next, paramagnetic microspheres (not shown) can be introduced at the upper end of capillary 14. The solenoid 22 is moved to the upper end of capillary 14 by means of the moving device 28. The paramagnetic means will be attracted to the area of maximum magnetic field strength, i.e. to the center of solenoid 22.

Then, when solenoid 22 is moved along the longitudinal direction of the capillary 14 by means of moving device 28, the microspheres will remain in the area of maximum magnetic field strength and thus follow the movement of the solenoid 22.

The solenoid 22 is then moved by the moving device 28 under control of control unit 16 such as to expose the microspheres consecutively with each of liquids 30c, 30b and 30a for a predetermined time, thereby processing the surface of the microspheres (not shown).

While only three different liquids 30a to 30c have been shown in Fig. 1 for simplicity, it is understood that the apparatus 10 and the method described are not limited to this. Consequently, the microspheres can be moved easily and in a controlled manner through different liquids with only very little technical effort. This solution thus compares favourably to an ordinary design, where the microspheres would have to be transferred between different containers containing different liquids.

While the example of Figs. 1 and 2 have been described with reference to functionalizing microspheres for immunoassays, the invention is not limited to this application. Instead, the invention may find use in any application where some type of object or carrier is to be exposed to different liquids consecutively, and will therefore find many useful applications in biochemical, medical or pharmaceutical laboratories.

Although a preferred exemplary is shown and specified in detail in the drawings and the preceding specification, these should be viewed as purely exemplary and not as limiting the invention. It is noted in this regard that only the preferred exemplary embodiment is shown and specified, and all variations and modifications should be protected that presently or in the future lie within the scope of protection as set forth in the appendant claims.

### Reference numerals

- 10: apparatus for bringing a carrier and spheres in contact consecutively with at least two liquids
- 12: filling means
- 14: capillary
- 16: control unit
- 18: signal line
- 20: pressurizing device
- 22: solenoid
- 24: bore of solenoid
- 26: magnetic field lines
- 28: moving device for solenoid 22
- 30a-30c: liquids
- 32: trapped gas volume

## Claims

1. A method of stacking at least two different liquids (30a- 30c) in a microchannel or capillary (14), comprising the following steps:
- filling a first liquid (30a) and a second liquid (30b) into the microchannel or capillary (14), and
- removing a gas volume (32) trapped between said first and second liquids (30a, 30b) by:
- exposing said gas volume (32) to a force biasing said gas volume along the longitudinal axis of the microchannel or capillary (14), and
- generating a pressure inside said microchannel or capillary (14) suitable to compress said gas volume (32) such that said compressed gas volume (32) is moved through one of said first and second liquids (30a, 30b) in response to said force.

2. The method of claim 1, wherein said force biasing said gas volume (32) along the longitudinal axis is a hydrostatic lift.

3. The method of claim 1 or 2, wherein at least one of said first and second liquids (30a, 30b) comprises a solution containing receptors for use in biochemical, medical or pharmaceutical assays, and in particular antigens or antibodies for use in immunoassays.

4. The method of one of the preceding claims, wherein at least one of said first and second liquids (30a, 30b) comprises an analyte for detection in biochemical, medial or pharmaceutical assays and/or
wherein at least one of said first and second liquids comprises a washing solution or a solution comprising conjugate markers.

5. The method of one of the preceding claims, wherein the microchannel or capillary (14) has an inner diameter of 300 µm - 1 mm, preferably 400 µm - 800 µm and/or wherein the longitudinal length of the volume of the first and/or second liquid (30a, 30b) is at least 1, preferably at least 5 mm.

6. The method of one of the preceding claims, wherein the pressure generated inside said microchannel or capillary (14) is between 0.5 and 1 MPa.

7. A method of bringing a carrier, and in particular a microbead or microsphere in surface contact with at least two different liquids (30a - 30c), said method comprising the following steps:
stacking at least two different liquids (30a - 30c) in a microchannel or capillary (14),
preferably according to one of claims 1 to 6, and
moving said carrier along the longitudinal axis of said microchannel or capillary (14) through said at least two liquids (30a - 30c).

8. The method of claim 7, wherein said liquids (30a - 30c) are stationary within said microchannel or capillary (14).

9. The method of one of claims 7 or 8, wherein said movement of said carrier along the axis of said microchannel or capillary (14) is controlled by applying a force to said carrier, and in particular by applying one or more of an electromagnetic force, an electrophoretic force, a magnetic force or a force obtained by optical tweezers.

10. The method of claim 9, wherein said carrier comprises a paramagnetic material and/or wherein said electromagnetic force on said carrier is generated by means of a magnetic field which has a local maximum of field strength inside said microchannel or capillary (14) and which is movable with respect to the microchannel or capillary (14) along the longitudinal axis thereof.

11. The method of one of claims 7 to 10, wherein the velocity of said movement of said carrier with respect to said liquids (30a- 30c) is controlled such that the surface contact to each of said liquids (30a - 30c) is sustained for predefined times.

12. An apparatus (10) for bringing a carrier in surface contact consecutively with at least two liquids (30a - 30c) comprising:
- means (12) for filling a first liquid (30a) and a second liquid (30b) into a microchannel or capillary (14),
- means (20) for generating a pressure inside said microchannel or capillary (14) suitable for compressing a gas volume (32) trapped between said first and second liquids (30a, 30b) such that said compressed gas volume (32) is moved through one of said at least two liquids (30a, 30b), and
- means for applying a force to said carrier such as to move said carrier through said at least two liquids (30a - 30c).

13. The apparatus (10) of claim 12, said apparatus further comprising a control unit (16) for carrying out a method according to one of claims 1 to 11.

14. The apparatus (10) of claim 12 or 13, wherein the means (22) for applying a force to said carrier comprises a solenoid (22) adapted to receive an electrical current and containing a bore (24) in which the microchannel or capillary (14) is received, wherein said solenoid (22) is moveable in longitudinal direction of said microchannel or capillary (14).
